# EUROPEAN PATENT APPLICATION

(11) **EP 4 497 373 A1**
(43) Date of publication of application: **29.01.2025**
(21) Application number: 23773424.9
(22) Date of filing: 22.03.2023
(51) Int. Cl.: A61B 5/00, A61B 5/01, A61B 5/02, A61B 5/08, A61B 5/11, A61B 5/024, A61B 5/0205

(54) **SYSTEM FOR OBTAINING BIOMETRIC PARAMETERS BY MEANS OF A COLLAR-MASK DEVICE**

(30) Priority: 23.03.2022 CL 20220721
(71) Applicant: Universidad de Talca, Talca (CL)
(72) Inventor: GATICA ROJAS, Valeska Fabiola, Condominio Estancia Las Rastras, Talca (CL); IBARRA CORTÉS, Natalia Audoriza, Talca (CL); GONZÁLEZ MARTÍNEZ, Luis Eduardo, Puertas del Sur, Talca (CL); ASTUDILLO HERNÁNDEZ, César Alejandro, Curicó (CL); AYALA AVILÉS, Jorge Eduardo, Maule (CL)
(74) Representative: Mendigutía Gómez, Maria Manuela
(86) International application number: PCT/CL2023/050025
(87) International publication number: WO 2023/178458

(57) **Abstract**

The present invention relates to the field of health and physical rehabilitation. In particular, the present invention provides a system, a method, and a device for obtaining biometric parameters by means of a device having sensors, connected to an electronic device, thus providing a smart tool that helps preventing the physical sequela of prevalent respiratory diseases, improving the level of function, independence, and quality of life of senior adults. The system includes a collar-mask type device to be worn by an individual, which is composed of a textile material and multiple sensors included in said textile material; and a computer application that is connected to said multiple sensors positioned in the collar-mask type device by means of a wireless connection with said electronic device.

## Description

### TECHNICAL FIELD OF INVENTION

The present invention relates to the field of medicine or veterinary science and hygiene, specifically to the field of measurement for diagnostic purposes, for multiple sensor units connected to the patient using a body or personal area network. In particular, it provides a system for obtaining biometric parameters using a collar-mask device connected to an electronic device, such as a smartphone.

### SUMMARY OF THE INVENTION

In the field of health, an important part of the field is the monitoring of patients by means of invasive or non-invasive sensors, said monitoring is used to prevent or diagnose some type of disease or pathology, especially in older patients.

Currently, there are several devices that help controlling parameters to maintain a good state of health, but they only focus on improving respiratory physiological aspects, and often contain peripheral systems that increase evaluation times.

An example of this type of monitoring currently being performed on patients is the invention disclosed in document US2021085255A1, wherein a method is described for using compressed physiological signal data to detect a health status of a user of the wearable device. The method includes producing, by a software application run on the wearable device, physiological signal data based on data recorded using one or more sensors of the wearable device. The physiological signal data indicates a physical quality of the user of the wearable device. The software application compresses the physiological signal data using a "dictionary" defined by a server application run on a server device. The physiological signal data is communicated from the wearable device to a server. The server application decompresses the physiological signal data to produce physiological signal data generated by the user indicating the physical quality of the user of the wearable device. The server application determines a change in the physiological state of the user of the wearable device based on a difference between the physical quality of the user of the wearable device indicated by the generated physiological signal data and the historical physiological data of the user of the wearable device. The server application detects the health condition of the user of the wearable device based on the change in the physiological state of the user of the wearable device.

On the other hand, document US2020297243A1 proposes a system that estimates the motion of a person or the like, the system comprising: a sensor that is attached to the waist of the person and measures various parameters that vary with a motion of the person, said parameters are measured by sensors in a time series; and a motion estimating unit configured to estimate position values indicating positions at a determined time of a plurality of predetermined regions of a body of the person. The motion estimation is configured to learn in accordance with an algorithm of a machine learning model, such that the reference position values of the plurality of regions of the body of the person are output at an answer time.

As can be appreciated, the aforementioned documents focus on improving respiratory physiological aspects without providing the right dose that would be required from it, depending on the physical condition of the individual. Moreover, they ignore the focus on motivation, adherence, and the performance of physical exercises in an interactive and entertaining way. Consequently, there is a need to create monitoring and control systems that are more accurate and complete, and that allow preventing, timely detecting, educating, and treating physical sequela of respiratory diseases prevalent in individuals.

### SUMMARY OF THE INVENTION

The present invention provides a system for obtaining biometric parameters in an individual by means of a device having multiple sensors connected to an electronic device, characterized in that it comprises: a collar-mask type device to be worn by said individual, wherein the collar-mask device is composed of a textile material and multiple sensors included in said textile material; and a computer application containing an interface that connects to said multiple sensors located in the collar-mask type device via a wireless connection.

In a preferred embodiment, the system is characterized in that the computer application, by means of a mathematical algorithm, determines at least one biometric parameter of said individual, wherein at least one biometric parameter is a physiological indicator.

In another preferred embodiment, the system is characterized in that the computer application, by means of a mathematical algorithm, determines at least one biometric parameter, wherein said biometric parameter relates to variations in the movement of the individual.

In a further preferred embodiment, the system is characterized in that said collar-mask type device contains two 3D inertial sensors, a respiratory rate and gas sensor, and an integrated heart rate, body temperature, and blood oxygenation sensor.

In another preferred embodiment, the system is characterized in that, when said device is placed on the individual, a first inertial sensor is positioned on the manubrium of the sternum of the individual, and a second inertial sensor is positioned on the spinous process of the seventh cervical vertebra of said individual.

In a preferred embodiment, the system is characterized in that the respiratory rate and gas sensor is positioned on the projection of and lateral to the corner of the mouth of said individual, while the integrated heart rate, body temperature, and blood oxygenation sensor is positioned on the projection of the external carotid artery or in the earlobe area of said individual.

In another preferred embodiment, the system is characterized in that said physiological indicator is selected from the group consisting of heart rate, respiratory gases, blood oxygenation percentage, body temperature, and respiratory rate.

In a preferred embodiment, the system is characterized in that said biometric parameter that relates to variations in the movement of the individual is selected from the group consisting of distance walked, translation speed, translation acceleration, center of mass (COM), and posture angle.

In another preferred embodiment of the invention, the electronic device that connects to said collar-mask type device by means of a computer application via a wireless connection is a mobile device, most preferably a smartphone.

In a further preferred embodiment, the collar-mask type device is internally composed of multiple layers of fabrics, metal mesh, and plastic masks.

The present invention further provides a method for obtaining biometric parameters by means of a device having multiple sensors connected to an electronic device, characterized in that it comprises: providing a collar-mask type device to be worn by an individual, the devices composed of a textile material and multiple sensors included or located in said textile material; locating each sensor of the collar-mask type device on specific areas of the body of said individual; connecting a computer application containing an interface that connects to said sensors located in the collar-mask type device via a wireless connection; performing some physical activity so that said sensors detect biometric parameters of interest; obtaining and analyzing from said computer application the information captured by the sensors related to said physical activity; and delivering results on the biometric parameters evaluated.

In a preferred embodiment, the method is characterized in that said computer application, by means of a mathematical algorithm, determines a biometric parameter of said individual, wherein said biometric parameter is a physiological indicator.

In another preferred embodiment, the method is characterized in that said computer application, by means of a mathematical algorithm, determines a biometric parameter, wherein said biometric parameter relates to variations in the movement of the individual.

In a further preferred embodiment, the method is characterized in that a first sensor (3) is positioned on the manubrium of the sternum of the individual, a second sensor (4) is positioned on the spinous process of the cervical vertebra of said individual, a third sensor (1) is positioned on the projection of the alar cartilages, and a fourth sensor (2) is positioned on the projection of the external carotid artery or in the earlobe area of said individual.

In another preferred embodiment, the method is characterized in that said physiological indicator is selected from the group consisting of heart rate, respiratory gases, blood oxygenation percentage, body temperature, and respiratory rate.

In a preferred embodiment, the method is characterized in that said biometric parameter that relates to variations in the movement of the individual is selected from the group consisting of distance walked, translation speed, translation acceleration, center of mass (COM), and posture angle.

In another preferred embodiment, the electronic device that connects to said sensors located on the collar-mask device via a wireless connection by means of said computer application, is a mobile device, most preferably a smartphone.

The present invention further relates to a device for obtaining biometric parameters by means of sensors on an individual, characterized in that it comprises a collar-mask type device composed of a textile material and multiple sensors included in said textile material and connected to an electronic device, which is preferably a smartphone.

### BRIEF DESCRIPTION OF THE FIGURES

FIG. 1 illustrates a representative image of the collar-mask type device of the invention, showing the location of the different sensors included therein, wherein 1 is a respiratory rate and gas sensor; 2 is an integrated heart rate, body temperature, and blood oxygenation sensor; 3 is an inertial sensor 1; and 4 is an inertial sensor 2.
FIG. 2 illustrates a representative image of the interior of the collar-mask type device in an orthogonal perspective, wherein the inner layers that comprise it are shown joined together.
FIG. 3 illustrates a representative image of the interior of the collar-mask type device from a left lateral perspective, wherein the inner layers are shown joined together.
FIG. 4 illustrates a representative image of the interior of the collar-mask type device from a right side perspective, wherein the inner layers of said device are shown separately.

### DETAILED DESCRIPTION OF THE INVENTION

The present invention discloses a system and a method that offers a technological tool that includes a collar-mask having multiple sensors, which is connected to a computer application, the use thereof focused on non-fragile or fragile elderly people, as well as the use by health professionals and caregivers of elderly people to prevent, detect early, educate, and treat physical sequela in prevalent respiratory or confinement diseases. Said system and method offer to improve respiratory physiological and postural control indicators through telerehabilitation with 2D virtual environments and telemonitoring for the control of biometric parameters in general, and in particular of physiological and posture-related indicators. This technology is preferably implemented as a highly functional and ergonomic collar-mask type sports model, it is also a mask developed to perform physical exercises outdoors or in a gym-type space.

In particular, the system for obtaining biometric parameters by means of a device having multiple sensors is connected to an electronic device, and is characterized in that it comprises: a collar-mask type device to be worn by an individual, said device being composed of a textile material and multiple sensors included in said textile material; and a computer application containing an interface that connects to said multiple sensors located in the collar-mask type device via a wireless connection, wherein the electronic device is a mobile device, most preferably a smartphone.

In the context of the present invention, and by way of general clarification throughout the description, a collar-mask device will be understood as a piece of clothing that covers at least the nose, mouth, and neck of an individual, which is made of a textile material, and wherein a plurality of sensors is placed covering the areas from which the desired parameters of interest are obtained. In additional embodiments of the invention, the collar-mask device may also cover the ears and chest.

In the context of the present invention, and by way of general clarification throughout the description, biometric parameters will be understood as any parameter referring to the physical, physiological, or postural characteristics of an individual.

In the context of the present invention, and by way of general clarification throughout the description, physiological indicators will be understood as any value of variables that allow the proper functioning of the biological systems of an individual.

In a preferred embodiment, the system is characterized in that the computer application, by means of a mathematical algorithm, determines a biometric parameter of said individual, wherein said biometric parameter is a physiological indicator.

In another preferred embodiment, the system is characterized in that the computer application, by means of a mathematical algorithm, determines a biometric parameter, wherein said biometric parameter relates to variations in the movement of the individual.

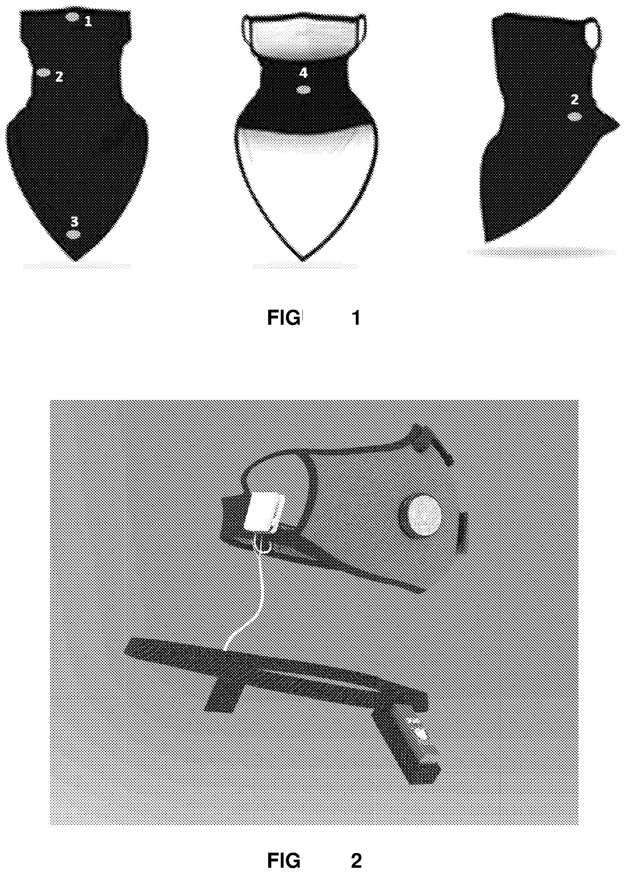

In a further preferred embodiment, the system is characterized in that said collar-mask type device contains two 3D inertial sensors (FIG. 1: point **3,** which is positioned on the manubrium of the sternum of the individual, and point **4,** which is positioned on the spinous process of the cervical vertebra of said individual, on the back of the collar-mask device), an integrated heart rate, body temperature, and blood oxygenation sensor (FIG. 1: point **2),** and a respiratory rate and gas sensor (FIG. 1: point **1).**

In another preferred embodiment, the system is characterized in that a first inertial sensor is positioned on the manubrium of the sternum of the individual, and a second inertial sensor is positioned on the spinous process of the seventh cervical vertebra of said individual.

In a preferred embodiment, the system is characterized in that the respiratory rate and gas sensor is positioned on the projection and lateral to the corner of the mouth of said individual.

In a further preferred embodiment, the system is characterized in that the integrated heart rate, body temperature, and blood oxygenation sensor is positioned on the projection of the external carotid or earlobe area of said individual.

In another preferred embodiment, the system is characterized in that said physiological indicator is selected from the group consisting of heart rate, respiratory gases, blood oxygenation percentage, body temperature, and respiratory rate.

In a preferred embodiment, the system is characterized in that said biometric parameter that relates to variations in the movement of the individual is selected from the group consisting of distance walked, translation speed, translation acceleration, center of mass (COM), and posture angle.

In a preferred embodiment, the system is characterized in that the computer application delivers a diagnostic report via web or email.

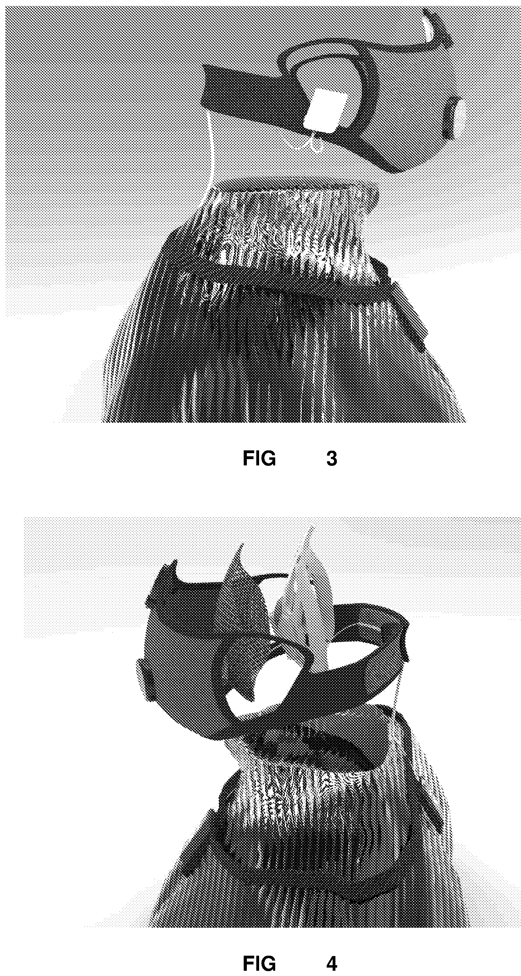

In a further preferred embodiment, the collar-mask type device is internally composed of multiple layers in which the multiple sensors are located, wherein said layers are composed of a first layer consisting of a fabric where the respiratory sensors are located, a plastic mask that provides rigidity to the collar-mask type device and that is placed before the layer of fabric containing the respiratory sensors, and a layer that is a metal mesh that separates both layers (FIG. 4). Although this is a preferred embodiment of the invention, variants of this device having these and other layers can be envisioned and used to measure the biometric parameters required, without departing from the desired scope of protection.

The computer application software will deliver a visual report on the screen in non-technical language in the case of the elderly (AM, by its Spanish acronym) or caregivers, and in technical words in the case of health professionals and rehabilitators. In either case, the report provided can be stored on the web and sent via email, or in PDF format to be printed on paper.

This technology can be used before, during, and at the end of physical exercise, or when performing a daily life activity such as walking. Therefore, it will be able to evaluate physiological indicators in this regard: body posture by means of a mathematical algorithm, heart rate (beats/minutes), respiratory gases, blood oxygenation percentage, body temperature, and respiratory rate (cycles/minutes), which are key parameters in the elderly population and which are directly affected by prevalent diseases such as flu, influenza, pneumonia, Covid-19, among others, and by the confinement of this population, caused for example, by the latter disease. On the other hand, the percentage of oxygenation in blood is a measure that has assumed a value in the current times of pandemic, specially, to detect at an early stage the silent hypoxemia caused by Covid-19.

Another object of the present invention is a method for obtaining biometric parameters by means of a device having multiple sensors connected to an electronic device, characterized in that it comprises: providing a collar-mask type device to be worn by an individual, the device composed of a textile material and multiple sensors included in said textile material; locating each sensor of the collar-mask type device in specific areas of the body of said individual; connecting a computer application containing an interface that connects to said sensors located in the collar-mask type device via a wireless connection; performing some physical activity so that said sensors detect biometric parameters of interest; obtaining and analyzing, from said computer application, the information captured by the sensors related to said physical activity; and delivering results on the biometric parameters evaluated in said individual.

In a preferred embodiment, the method is characterized in that said computer application, by means of a mathematical algorithm, determines a biometric parameter of said individual, wherein said biometric parameter is a physiological indicator.

In another preferred embodiment, the method is characterized in that said computer application, by means of a mathematical algorithm, determines a biometric parameter, wherein said biometric parameter is related to variations in the movement of the individual.

In a further preferred embodiment, the method is characterized in that a first inertial sensor **(3)** is positioned on the manubrium of the sternum of the individual, a second inertial sensor **(4)** is positioned on the spinous process of the cervical vertebra of said individual, a third sensor **(1)** is positioned on the projection of the alar cartilages, and a fourth sensor **(2)** is positioned on the projection of the external carotid artery or earlobe area of said individual.

In another preferred embodiment, the method is characterized in that said physiological indicator is selected from the group consisting of heart rate, respiratory gases, blood oxygenation percentage, body temperature, and respiratory rate.

In a preferred embodiment, the method is characterized in that said biometric parameter that relates to variations in the movement of the individual is selected from the group consisting of distance walked, translation speed, translation acceleration, center of mass (COM), and posture angle.

The design of this product will allow solving the proposed problem through scientific and technological integration, contributing to the creation of a smart tool, preventing the physical sequela of prevalent respiratory diseases, improving the functionality, independence, and quality of life of the elderly.

## Claims

1. A system for obtaining biometric parameters of an individual by means of a device having sensors connected to an electronic device, **CHARACTERIZED in that** it comprises:
- a collar-mask type device to be worn by an individual, including multiple sensors located at the height between the nose and the sternum of the individual, and included in a textile material; and
- a computer application that connects to said multiple sensors located in the collar-mask type device via a wireless connection with said electronic device.

2. The system of claim 1, **CHARACTERIZED in that** the collar-mask type device includes internally multiple layers wherein said multiple sensors comprising it are placed.

3. The system of claim 2, **CHARACTERIZED in that** said multiple layers are selected from the group consisting of layers of fabrics, metal mesh, and plastic masks.

4. The system of claim 1, **CHARACTERIZED in that** the computer application, by means of a mathematical algorithm, determines a biometric parameter of said individual, wherein said biometric parameter is a physiological indicator.

5. The system of claim 1, **CHARACTERIZED in that** the computer application, by means of a mathematical algorithm, determines a biometric parameter, wherein said biometric parameter relates to variations in movement of the individual.

6. The system of any one of claims 1 to 5, **CHARACTERIZED in that** said collar-mask type device contains two 3D inertial sensors, an integrated heart rate, body temperature, and blood oxygenation sensor, and a respiratory rate and gas sensor.

7. The system of claim 6, **CHARACTERIZED in that**, in the collar-mask type device, a first inertial sensor (3) is positioned at the height of the manubrium of the sternum of the individual, and a second inertial sensor (4) is positioned at the height of the spinous process of the seventh cervical vertebra of said individual, the respiratory frequency and gas sensor (1) is positioned at the height of the projection of the alar cartilages and lateral to the corner of the mouth of said individual, and the integrated heart rate, body temperature, and blood oxygenation sensor (2) is positioned at the height of the projection of the external carotid artery or in the earlobe area of said individual.

8. The system of claim 2, **CHARACTERIZED in that** said physiological indicator is selected from the group consisting of heart rate, respiratory gases, blood oxygenation percentage, body temperature, and respiratory rate.

9. The system of claim 3, **CHARACTERIZED in that** said biometric parameter that relates to variations in movement of the individual is selected from the group consisting of distance walked, translation speed, translation acceleration, center of mass (COM), and posture angle.

10. The system of any one of claims 1 to 9, **CHARACTERIZED in that** the electronic device that connects to the sensors of said collar-mask type device by means of a computer application via a wireless connection is a mobile device.

11. The system of claim 10, **CHARACTERIZED in that** said mobile device is a smartphone.

12. A method for obtaining biometric parameters of an individual by means of a device having multiple sensors connected to an electronic device, **CHARACTERIZED in that** it comprises:
- providing a collar-mask type device to be worn by the individual, including multiple sensors located at a height between the nose and the sternum of the individual, which are included in a textile material;
- locating each sensor of the collar-mask type device on specific areas of the body of said individual;
- connecting, via a wireless connection by means of a computer application, the multiple sensors of the collar-mask type device to an electronic device;
- performing some physical activity by the individual so that said sensors detect biometric parameters of interest;
- obtaining and analyzing, from said computer application, the information captured by the sensors related to the physical activity of the individual; and
- delivering results on the biometric parameters evaluated.

13. The method of claim 12, **CHARACTERIZED in that** said computer application, by means of a mathematical algorithm, determines a biometric parameter of said individual, wherein said biometric parameter is a physiological indicator.

14. The method of claim 12, **CHARACTERIZED in that** said computer application, by means of a mathematical algorithm, determines a biometric parameter, wherein said biometric parameter relates to variations in movement of the individual.

15. The method of claim 14, **CHARACTERIZED in that** a first inertial sensor (3) of the device is positioned at the height of the manubrium of the sternum of the individual, a second inertial sensor (4) of the device is positioned at the height of the spinous process of the seventh cervical vertebra of said individual, a third sensor (1) of the device is positioned at the height of the projection of the alar cartilages, and a fourth sensor (2) of the device is positioned at the height of the projection of the external carotid artery or in the earlobe area of said individual.

16. The method of claim 13, **CHARACTERIZED in that** said physiological indicator is selected from the group consisting of heart rate, respiratory gases, blood oxygenation percentage, body temperature, and respiratory rate.

17. The method of claim 14, **CHARACTERIZED in that** said biometric parameter that relates to variations in the movement of the individual is selected from the group consisting of distance walked, translation speed, translation acceleration, center of mass (COM), and posture angle.

18. The method of claim 12, **CHARACTERIZED in that** the electronic device that connects to the sensors of said collar-mask type device by means of a computer application via a wireless connection is a mobile device.

19. The method of claim 18, **CHARACTERIZED in that** said mobile device is a smartphone.

20. A device for obtaining biometric parameters of an individual by means of sensors, **CHARACTERIZED in that** it includes multiple sensors positioned at the height between the nose and the sternum of the individual, which are included in a textile material, said sensors being connected to an electronic device.

21. The device of claim 20, **CHARACTERIZED in that** it includes internally multiple layers wherein said multiple sensors comprising it are placed.

22. The device of claim 21, **CHARACTERIZED in that** said multiple layers are selected from the group consisting of layers of fabric, metal mesh, and plastic masks.

23. The device of any one of claims 20 to 22, **CHARACTERIZED in that** it includes two 3D inertial sensors, an integrated heart rate, body temperature, and blood oxygenation sensor, and a respiration rate sensor.

24. The device of claim 23, **CHARACTERIZED in that** a first inertial sensor (3) is positioned at the height of the manubrium of the sternum of the individual, a second inertial sensor (4) is positioned the height of the spinous process of the seventh cervical vertebra of said individual, the respiratory rate and gas sensor (1) is positioned at the height of the projection of the alar cartilages and lateral to the corner of the mouth of said individual, and the integrated heart rate, body temperature, and blood oxygenation sensor (2) is positioned at the height of the projection of the external carotid artery or in the earlobe area of said individual.

25. The device according to claim 20, **CHARACTERIZED in that** the electronic device to which the multiple sensors are connected is a smartphone.
